# EUROPEAN PATENT APPLICATION

(11) **EP 4 111 954 A1**
(43) Date of publication of application: **04.01.2023**
(21) Application number: 21183337.1
(22) Date of filing: 02.07.2021
(51) Int. Cl.: A61B 5/026, A61B 5/024, A61B 5/00

(54) **GENERATING A REPRESENTATION OF TISSUE FROM AN AMBIENT LIGHT CORRECTED IMAGE**

(71) Applicant: Koninklijke Philips N.V., 5656 AG Eindhoven (NL)
(72) Inventor: Damodaran, Mathivanan, 5656 AE Eindhoven (NL); Palero, Jonathan Alambra, 5656 AE Eindhoven (NL); Bourquin, Yannyk Parulian Julian, 5656 AE Eindhoven (NL)
(74) Representative: Philips Intellectual Property & Standards

(57) **Abstract**

According to an aspect, there is provided a system (100) for generating image data comprising: a light source (104) configured to provide temporally modulated illumination to tissue of a subject; an imaging unit (106) configured to capture, while the tissue is illuminated by the temporally modulated illumination, a plurality of images of the tissue of the subject, wherein each of the plurality of images includes a plurality of bright bands and dark bands, wherein a bright band corresponds to a high state of the temporally modulated illumination and a dark band corresponds to a low state of the temporally modulated illumination; and a processor (102) configured to: generate a composite image based on the plurality of images or a subset of the plurality of images; generate an ambient light corrected image based on the plurality of images; measure, using the ambient light corrected image, a parameter of the tissue; generate a representation of the tissue based on a combination of the composite image and an indication of the measured parameter; and provide the generated representation for display.

## Description

### FIELD OF THE INVENTION

The invention relates to generating an image for display, using an image that has undergone ambient light correction processing.

### BACKGROUND OF THE INVENTION

There is a drive towards innovation in the field of non-obtrusive measurement and monitoring, particularly in the field of personal healthcare. An example of an application in this field is skin sensing or skin/tissue monitoring, which enables analysis of a person's skin or tissue in a non-obtrusive way. Systems used for skin monitoring may enable a user to detect features or obtain information about features that are too small or otherwise undetectable to the naked eye.

Whether skin monitoring is performed in a medical environment or in a home environment, ambient lighting in the vicinity of the person whose skin is being analyzed may vary and this could give rise to inconsistent outputs from an imaging device used to image the skin. Thus, there is a desire to improve the robustness of such systems, to enable more accurate visualization and analysis of skin in an environment where ambient lighting conditions are varying.

### SUMMARY OF THE INVENTION

There is a need for an improved mechanism for enabling a person's tissue to be monitored and, specifically, for enabling the images to be generated that can be used for accurate analysis of the subject's tissue and also for improved visualization of the subject's tissue. The inventors of the present disclosure have recognized that accurate analysis of tissue can be performed using an image that has been processed to correct for varying ambient light conditions. The inventors have also recognized that visualization of the analysis of the tissue is improved if the results of the analysis are presented on an image of the tissue that has not been processed to correct for the varying ambient light conditions. In this way, a subject is able to visualize a measured or analyzed parameter of their tissue on an image or representation that does not appear modified.

According to a first specific aspect, there is provided a system for generating image data, the system comprising a light source configured to provide temporally modulated illumination to tissue of a subject; an imaging unit configured to capture, while the tissue is illuminated by the temporally modulated illumination, a plurality of images of the tissue of the subject, wherein each of the plurality of images includes a plurality of bright bands and dark bands, wherein a bright band corresponds to a high state of the temporally modulated illumination and a dark band corresponds to a low state of the temporally modulated illumination; and a processor configured to: generate a composite image based on the plurality of images or a subset of the plurality of images generate an ambient light corrected image based on the plurality of images; measure, using the ambient light corrected image, a parameter of the tissue; generate a representation of the tissue based on a combination of the composite image and an indication of the measured parameter; and provide the generated representation for display.

The imaging unit may be configured to operate in a rolling shutter mode. The light source may be configured to provide temporally modulated illumination in the form of at least one temporally modulated illumination signal having a wavelength falling within a defined waveband. A frequency of the temporally modulated illumination may not be a multiple integral of a frame rate of the imaging unit.

In some embodiments, the system may further comprise a display unit configured to display at least one of: the plurality images; the composite image; the ambient light corrected image; an indication of the measured parameter; and the generated representation.

The light source may, in some embodiments, be configured to generate temporally modulated illumination having a wavelength within a defined waveband. The waveband may be selected based on at least one of: an imaging parameter of the imaging unit used to capture the plurality of images; and an indication of the parameter of the tissue that is to be measured.

The processor is configured to measure a parameter of the tissue by extracting, from the ambient light corrected image, data corresponding to at least one of a red image channel, a green image channel and a blue image channel; and determining a physiological parameter of the tissue from at least one of the red image channel, the green image channel and the blue image channel.

According to a second specific aspect, there is provided an interactive mirror comprising a reflective component; and a system as disclosed herein.

According to a third specific aspect, there is provided a computer-implemented method for generating image data, the method comprising receiving a plurality of images of tissue of a subject illuminated by temporally modulated illumination, wherein each of the plurality of images includes a plurality of bright bands and dark bands, wherein a bright band corresponds to a high state of the pulsed illumination and a dark band corresponds to a low state of the temporally modulated illumination; generating a composite image based on the plurality of images or a subset of the plurality of images; generating an ambient light corrected image based on the plurality of images; measuring, using the ambient light corrected image, a parameter of the tissue; generating a representation of the tissue based on a combination of the composite image and an indication of the measured parameter; and providing the generated representation for display.

The method may further comprise generating, based the ambient light corrected image, image data representing the tissue in a color corresponding to a defined waveband. Measuring a parameter may comprise measuring a parameter using the image data.

In some embodiments, measuring the parameter of the tissue may comprise applying at least one filter to the ambient light corrected image to obtain at least one filtered ambient light corrected image; and determining quantitative information regarding the parameter from the at least one filtered ambient light corrected image.

The temporally modulated illumination may, in some embodiments, comprise temporally modulated illumination having a wavelength falling within a defined waveband.

The at least one defined waveband of the temporally modulated illumination may be selected based on a responsivity of an imaging unit used to capture the plurality of images. I other embodiments, the at least one defined waveband of the temporally modulated illumination may be selected based on an indication of the parameter of the tissue that is to be measured.

The method may further comprise applying gamma correction processing or inverse-gamma correction processing to at least one of: the plurality of images; the composite image; and the ambient light corrected image.

In some embodiments, generating a representation of the tissue may comprise generating, based on the measured parameter, a parameter image showing an indication of the measured parameter of the tissue; applying a weighting factor to the composite image and the parameter image; and generating a representation of the tissue based on a combination of the weighted composite image and the weighted parameter image.

According to a fourth specific aspect, there is provided a computer program product comprising a non-transitory computer-readable medium, the computer-readable medium having computer-readable code embodied therein, the computer-readable code being configured such that, on execution by a suitable computer or processor, the computer or processor is caused to perform steps of the method disclosed herein.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a schematic illustration of an example of a system for generating image data;
Fig. 2 is an illustration of various images generated using the system of Fig. 1;
Fig. 3 is a schematic illustration of a further example of a system for generating image data;
Fig. 4 is a schematic illustration of an example of an interactive mirror;
Fig. 5 is a flowchart of an example of a method for generating image data;
Fig. 6 is a flowchart of a further example of a method for generating image data; and
Fig. 7 is a schematic illustration of an example of a processor in communication with a computer-readable medium.

### DETAILED DESCRIPTION OF THE EMBODIMENTS

Embodiments of the present disclosure are described in terms of a system, an interactive mirror, a computer-implemented method and a computer program product. Various embodiments disclosed herein provide a mechanism by which a parameter of a subject's tissue may be measured using an ambient light corrected image of the tissue, and then an indication of the measured parameter may be presented on a non-ambient light corrected image of the tissue. In this way, accurate measurement of the parameter can take place using an image that has been modified to remove effects of ambient lighting conditions that could otherwise affect the measurement. The measured parameter can then be presented to a viewer (e.g. the subject whose tissue is being analyzed) on a version of the image that has not been modified to remove the effects of ambient lighting conditions, such that the viewer sees a representation of the tissue under the ambient lighting conditions present when the image was captured.

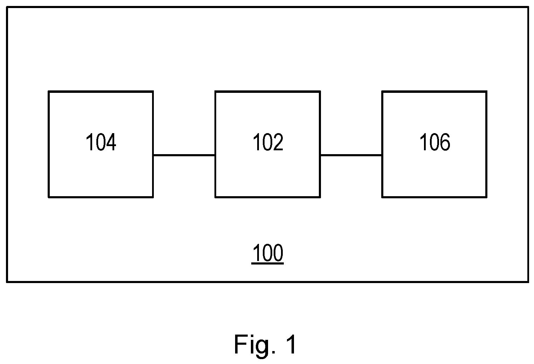

According to a first aspect, a system is provided. Referring to the drawings, Fig. 1 is a schematic illustration of an example of a system 100 for generating image data. In this example, components of the system 100 are shown as part of a single device or apparatus. However, the various components of the system 100 may form part of a distributed system, and may be connected or in communication with one another via one or more wired or wireless communication mechanisms.

The system 100 comprises a processor 102, a light source 104 and an imaging unit 106. The light source 104 is configured to provide illumination to tissue of a subject. For example, the illumination may be temporally modulated illumination. The illumination may be modulated according to any function (e.g. sinusoidally). In some embodiments, the light source 104 may be configured to generate pulsed illumination. The tissue of a subject that is to be illuminated may comprise a person's skin or any other tissue on a person's body, including part or all of their head, face, hand, foot, torso or the like. The light source 104 may, in some embodiments, comprise a light bulb or a light emitting diode (LED), and may be configured to generate light of a single wavelength, light having a range of wavelengths within a defined waveband, light having wavelengths within multiple wavebands and or white light/broadband illumination. The light source 104 may comprise a stand-alone light source, such as a lamp, or it may be incorporated into another device, such as a computing device (e.g. a smart phone or tablet computer) or an interactive mirror.

The imaging unit 106 is configured to capture a plurality of images of the tissue of the subject, while the tissue is illuminated by the temporally modulated illumination. The imaging unit 106 may, for example, comprise a camera device configured to capture a series of images or a series of frames of video footage. Each of the plurality of images includes a plurality of bright bands and dark bands, wherein a bright band corresponds to a high state of the temporally modulated illumination and a dark band corresponds to a low state of the temporally modulated illumination. The bands in the plurality of images may result, in some embodiments, from the use of an imaging unit 106 that is configured to employ a rolling shutter (e.g. a rolling shutter camera). In some embodiments, a relatively short camera exposure time may be used.

The processor 102 is in operable communication with the light source 104 and the imaging unit 106, either directly, or indirectly via other processing devices. In some embodiments, the processor 102 may be configured to operate the light source 104 generate the temporally modulated illumination and/or may be configured to operate the imaging unit to capture the plurality of images.

The processor 102 is configured to generate a composite image based on the plurality of images or a subset of the plurality of images. The plurality of images (or a subset thereof) may, for example, be combined using existing image processing techniques such that the temporally modulated illumination is extracted or demultiplexed from the (non-modulating) ambient light. The processor 102 is configured to generate an ambient light corrected image based on the plurality of images. Several techniques are known for processing images to correct for ambient light conditions, and the ambient light corrected image may be generated by the processor 102 using any suitable technique. Examples of two ambient light correction techniques are discussed below.

In a first example of an ambient light correction technique, the processor 102 may generate a first image by combining sections from the plurality of captured images which correspond to bright bands. The first image comprises a plurality of bright bands from the plurality of captured images. The processor 102 may then generate a second image by combining sections from the plurality of captured images which correspond to dark bands. The second image comprises a plurality of dark bands from the plurality of captured images. The processor 102 may then generate an ambient light corrected image based on a difference in pixel information between the first image and the second image. In some embodiments, the first image and the second image may be generated by applying an unmixing algorithm so as to separate pixels contained in the plurality of captured images on the basis of illumination state.

In a second example of an ambient light correction technique, the processor 102 may generate, for each of every pixel location in the plurality of images, a sorted list of pixels by sorting respective pixels each corresponding to the respective location in the plurality of images. The sorting is based on the pixel value of the respective pixels. For example, in the case the imaging unit is a monochrome camera, the pixel value is the pixel value captured by the imaging unit as is. As another example, in the case the imaging unit is an RGB camera, the pixel value may be an intensity value obtained through the processing of the R value, the G value and the B value of the respective pixel in the candidate image, e.g. using "Lab" color space and taking the L values. The sorting of respective pixels each corresponding to the respective location in the plurality of candidate images may be based on intensity. The processor may then apply, for each of every pixel location in the plurality of images, a set of weights to the respective sorted list of pixels, and to generate an estimated ambient light corrected image based on the plurality of weighted and sorted lists of pixels by summing the plurality of weighted and sorted lists of pixels. The processor 102 may determine the set of weights to be applied to the sorted lists of pixels based on an estimated signal-to-noise ratio (SNR) of the plurality of images. Alternatively, the processor 102 may determine the set of weights to be applied to the sorted lists of pixels based on at least one of: an exposure time at which the plurality of images are captured, a sensitivity level (ISO sensitivity) of the imaging unit, and a detected light level of the plurality of captured images.

Referring again to Fig. 1, the processor 102 is configured to measure, using the ambient light corrected image, a parameter of the tissue. Tissue parameters that may be measured include, but are not limited to, redness in tissue, oxygenation levels in tissue and inflammation of tissue. In other examples, tissue parameters that may be measured may include skin parameter maps indicative of blood perfusion, hydration, and/or levels of melanin, bilirubin, carotenoids and/or sebum. In some examples, a parameter or multiple parameters may be measured using the ambient light corrected image by performing appropriate image processing techniques and identifying/measuring image features (e.g. differences in color in adjacent pixels) in the processed image. In other examples, as discussed below, illumination of the tissue using light of a particular wavelength, or the application of one or more filters to the light or to the captured images, may enable a particular parameter to be measured and/or quantified.

The processor 102 is configured to generate a representation of the tissue based on a combination of the composite image and an indication of the measured parameter, and provide the generated representation for display. For example, the generated representation may be provided for display on a screen of a computing device (e.g. a smart phone, a tablet computer or an interactive mirror) that a user is using to view or monitor their tissue. The composite image generated based on the plurality of images (or a subset thereof) comprises an image of the tissue that has not been modified to compensate for the ambient light conditions. Thus, in an example where a subject is using a screen of a computing device (e.g. a smart phone, a tablet computer or an interactive mirror) to view an image of their tissue, the composite image is representative of how the subject would view their tissue on the screen of the computing device. Thus, by combining the composite image with an indication of the measured parameter, a viewer of the generated representation is able to clearly view the indication of the measured parameter on their tissue.

In some embodiments, the processor 102 may be configured to present the representation of the tissue in the form of a still image. In other embodiments, however, the processing performed by the processor 102 may be repeated such that new images are captured by the imaging unit 106 and processed in real-time, such that the representation of the tissue that is displayed to a viewer is updated effectively in real-time, with movement of the representation mirroring movement of the subject.

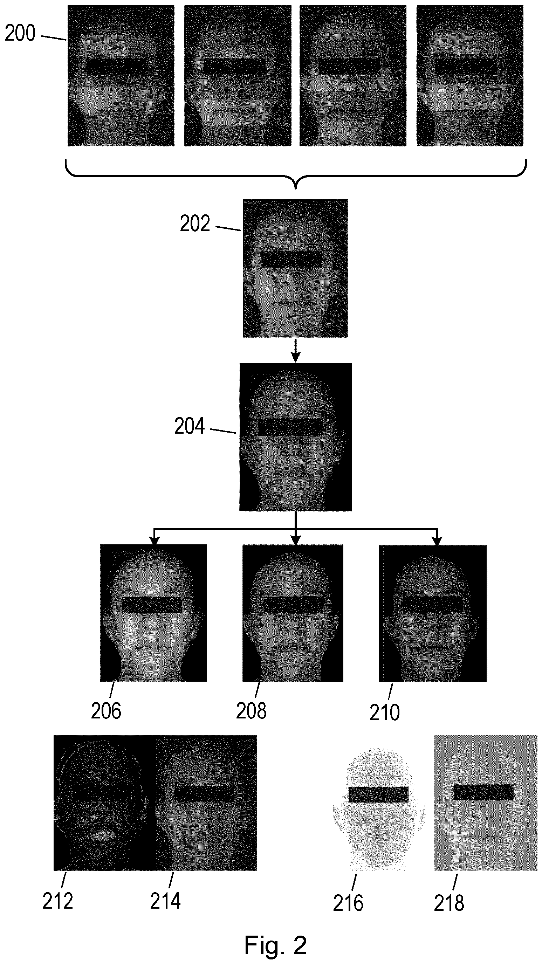

Fig. 2 is an illustration of various images generated using the system 100 discussed above, as the various processing tasks are performed using the processor 102. A plurality of images 200 are captured using the imaging unit 106. The images 200 include a plurality of bright bands and dark bands. In this example, the bright bands and the dark bands are shown to have drifted as the series of images have been captured. Such rolling bands may result from use of a rolling shutter camera. In examples where a rolling shutter camera is not used, the plurality of images may include a plurality of standing bands.

The plurality of images 200 are combined using existing image processing techniques to generate a composite image 202. For example, the bright bands from the plurality of images 200 may be combined to form a composite image 202 that includes just the bright bands. Image 204 is an ambient light corrected image generated based on the plurality of images 200. In the image 204, ambient light used to illuminate the tissue of the subject (e.g. light directed onto one side of the subject's face) is compensated for so that the lighting in the ambient light corrected image appears more uniform.

The ambient light corrected image 204 is then processed in order to measure a parameter of the tissue. In the example shown in Fig. 2, the ambient light corrected image 204 is processed to generate images from the red, green and blue channels: image 206 is formed from the red channel image data, image 208 is formed from the green channel image data and image 210 is formed from the blue channel image data. A different processed version of the ambient light corrected image 204 may be used depending on the tissue parameter to be measured.

Image 212 shows an example of an image formed using the red channel image 206 and the green channel image 210, which is used to analyze a level of oxygenation in the subject's tissue. A representation 214 (e.g. an image) has been created by combining image 212 (i.e. the indication of the measured parameter, oxygenation level) with the composite image 202 showing the subject tissue without any ambient light correction. The resulting representation 214 shows the subject's tissue in its original form (i.e. including the ambient lighting), with additional highlighting representing regions of high oxygenation levels. Thus, a viewer (e.g. the subject) of the resulting representation 214 is able to identify regions of high or low oxygenation in the tissue on a representation displayed to them. Without the techniques disclosed herein, such information about the tissue would not otherwise be readily available and visible to the human eye.

In another example, image 216 shows an example of an image formed using the red channel 206, which is used to analyze a level of redness in the subject tissue. In this example, however, a representation 218 has been created by combining image 216 (i.e. the indication of the measured parameter, redness) with the ambient light corrected image 204. The resulting representation 218 is therefore lacking much of the depth and detail that is apparent in the representation 214, and there would be available had the image 216 been combined with the composite image 202, which has not been processed for ambient light correction.

It is clear from the examples shown in Fig. 2 that, by presenting the indication of the measured parameter on the composite image that has not undergone ambient light correction, the resulting image shows a true representation of the subject's tissue, with the additional information about the measured parameter. Moreover, by performing ambient light correction on the image before analysis (e.g. before the parameter measurement is made), the parameter measurement can be made consistently, without being affected by the ambient light conditions.

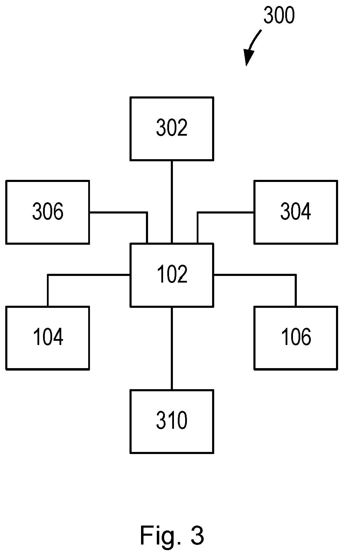

Fig. 3 is a schematic illustration of a further example of a system 300 for generating image data. In this example, the system 300 is represented as a distributed system, such that the components of the system are not part of a single unit or apparatus. In other embodiments, however, such as the embodiment shown in Fig. 1, the components may be part of a single apparatus. The system 300 shown in Fig. 3 includes the processor 102, the light source 104 and the imaging unit 106. The system 300 may further comprise a display unit 302 configured to display at least one of: the plurality images 200; the composite image 202; the ambient light corrected image 204; an indication of the measured parameter 212, 216; and the generated representation 214, 218. The display unit 302 may be configured to display other information.

In some embodiments, the system 300 may further comprise a memory 304 comprising instruction data representing a set of instructions. The memory 304 may be configured to store the instruction data in the form of program code that can be executed by the processor 102 to perform steps of the methods described herein. In some implementations, the instruction data can comprise a plurality of software and/or hardware modules that are each configured to perform, or are for performing, individual or multiple steps of the method described herein. In some embodiments, the memory 304 may be part of a device that also comprises one or more other components of the system 300 (for example, the processor 102 and/or one or more other components of the system). In alternative embodiments, the memory 304 may be part of a separate device to the other components of the system 300.

In some embodiments, the memory 304 may comprise a plurality of sub-memories, each sub-memory being capable of storing a piece of instruction data. In some embodiments where the memory 304 comprises a plurality of sub-memories, instruction data representing the set of instructions may be stored at a single sub-memory. In other embodiments where the memory 304 comprises a plurality of sub-memories, instruction data representing the set of instructions may be stored at multiple sub-memories. For example, at least one sub-memory may store instruction data representing at least one instruction of the set of instructions, while at least one other sub-memory may store instruction data representing at least one other instruction of the set of instructions. Thus, according to some embodiments, the instruction data representing different instructions may be stored at one or more different locations in the system 300. In some embodiments, the memory 304 may be used to store information, data (e.g. images), signals and measurements acquired or made by the processor 102 of the system 300 or from any other components of the system.

The processor 102 of the system 300 can be configured to communicate with the memory 304 to execute the set of instructions. The set of instructions, when executed by the processor 102 may cause the processor 102 to perform the method described herein. The processor 102 can comprise one or more processors, processing units, multi-core processors and/or modules that are configured or programmed to control the system 300 in the manner described herein. In some implementations, for example, the processor 102 may comprise a plurality of (for example, interoperated) processors, processing units, multi-core processors and/or modules configured for distributed processing. It will be appreciated by a person skilled in the art that such processors, processing units, multi-core processors and/or modules may be located in different locations and may perform different steps and/or different parts of a single step of the method described herein.

Returning again to Fig. 3, in some embodiments, the system 300 may comprise at least one user interface 306. In some embodiments, the user interface 306 may be part of a device that also comprises one or more other components of the system 300 (for example, the processor 102, the memory 304 and/or one or more other components of the system 300). In alternative embodiments, the user interface 306 may be part of a separate device to the other components of the system 300.

A user interface 306 may be for use in providing a user of the system 300 (for example, the subject or a medical professional analyzing the measured parameter of the tissue) with information (e.g. images) resulting from the method according to embodiments herein. The set of instructions, when executed by the processor 102 may cause processor to control one or more user interfaces 306 to provide information resulting from the method according to embodiments herein. Alternatively or in addition, a user interface 306 may be configured to receive a user input. In other words, a user interface 306 may allow a user of the system 300 to manually enter instructions, data, or information. The set of instructions, when executed by the processor 102 may cause the processor to acquire the user input from one or more user interfaces 306.

A user interface 306 may be any user interface that enables rendering (or output or display) of information, data or signals to a user of the system 300. Alternatively or in addition, a user interface 306 may be any user interface that enables a user of the system 300 to provide a user input, interact with and/or control the system. For example, the user interface 306 may comprise one or more switches, one or more buttons, a keypad, a keyboard, a mouse, a mouse wheel, a touch screen or an application (for example, on a tablet, smartphone or interactive mirror), a display screen, a graphical user interface (GUI) or other visual rendering component, one or more speakers, one or more microphones or any other audio component, one or more lights, a component for providing tactile feedback (e.g. a vibration function), or any other user interface, or combination of user interfaces.

In some embodiments, as illustrated in Fig. 3, the system 300 may also comprise a communications interface (or circuitry) 310 for enabling the system to communicate with interfaces, memories and/or devices that are part of the system. The communications interface 310 may communicate with any interfaces, memories and devices wirelessly or via a wired connection.

It will be appreciated that Figs. 1 and 3 only show the components required to illustrate this aspect of the disclosure and, in a practical implementation, the system 100, 300 may comprise additional components to those shown. For example, the system 100, 300 may comprise a battery or other power supply for powering the system 300 or means for connecting the system to a mains power supply.

As noted above, in some embodiments, the imaging unit 106 may be configured to operate in a rolling shutter mode. In such embodiments, the light source 104 may be configured to provide temporally modulated illumination in the form of at least one temporally modulated illumination signal having a wavelength falling within at least one defined waveband. The temporally modulated illumination may have a frequency that is not a multiple integral of the frame rate of the imaging unit 106. In some embodiments, a frequency of the temporally modulated illumination may be at least 60 Hz.

The light source 104 may be configured to generate temporally modulated illumination having a wavelength within a defined waveband; wherein the waveband is selected based on at least one of: an imaging parameter of the imaging unit 106 used to capture the plurality of images; and an indication of the parameter of the tissue that is to be measured. For example, a wavelength of the temporally modulated illumination may be selected to match a transmission window of a filter array of the imaging unit 106 in order to avoid crosstalk between channels of the imaging unit. In another example, the wavelength of the temporally modulated illumination may be selected because it is known that illumination of that particular wavelength enables a particular parameter of the tissue to be visualized or measured. As shown in Fig. 2, for example, redness in a person's tissue may be measured more readily when the tissue is illuminated with red light.

The processor 102 may measure a parameter of the tissue in the ambient light corrected image 204 using a number of existing techniques. In one example, the processor 102 may be configured to measure a parameter of the tissue by extracting, from the ambient light corrected image 204, data corresponding to at least one of a red image channel (e.g. Fig. 2; 206), a green image channel (e.g. Fig. 2; 208) and a blue image channel (e.g. Fig. 2; 210). The processor 102 may be configured to determine a physiological parameter of the tissue from at least one of the red image channel, the green image channel and the blue image channel. Such a physiological parameter may include a level of oxygenation, a level of redness and/or a level of inflammation, for example. In some examples, the processor 102 may be configured to determine a parameter of the tissue (e.g. a physiological parameter of the tissue) by performing ratiometric calculations on data acquired from the red, green, and/or blue channels. For example, calculating a ratio of the red channel and the green channel could be used to determine quantitative information relating to inflammation in the tissue.

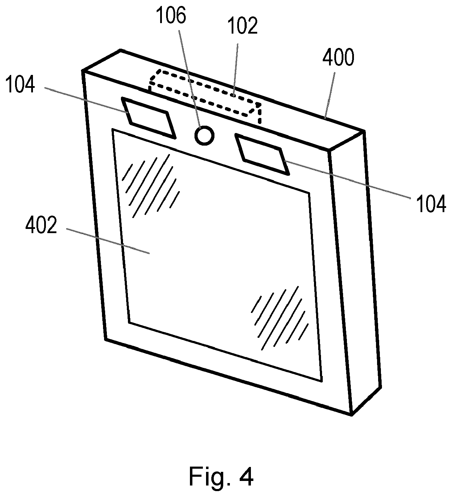

According to a second aspect, an interactive mirror is provided. Fig. 4 is an illustration of an example of an interactive mirror 400. The interactive mirror 400 comprises a reflective component 402 (e.g. a mirror component) and the system 100, 300 disclosed herein. For example, the interactive mirror 400 may comprise one or more light sources 104 and one or more imaging units 106 adjacent to the reflective component 402. The processor 102 may be located within a housing of the interactive mirror 400.

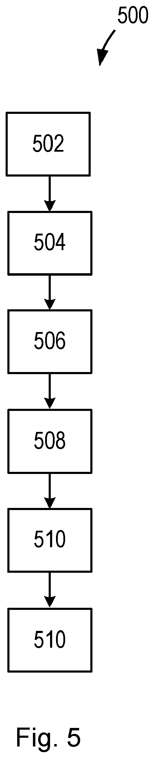

According to third aspect, a method is provided. Fig. 5 is a flowchart of an example of such a method 500. The method 500 may comprise a computer-implemented method for generating image data. In some embodiments, one or more steps of the method 500 may be carried out using the processor 102. The method 500 comprises, at step 502, receiving a plurality of images 200 of tissue of a subject illuminated by temporally modulated illumination, wherein each of the plurality of images includes a plurality of bright bands and dark bands, wherein a bright band corresponds to a high state of the pulsed illumination and a dark band corresponds to a low state of the temporally modulated illumination. The plurality of images 200 may, for example, be captured using the imaging unit 106 (e.g. a rolling shutter camera). Illumination of the issue of the subject may be provided by the light source 104.

In some embodiments, the temporally modulated illumination may comprise temporally modulated illumination having a wavelength falling within at least one defined waveband. The at least one defined waveband may be selected based on a property of the imaging unit used to capture the plurality of images. In some embodiments, for example, the at least one defined waveband of the temporally modulated illumination may be selected based on a responsivity of an imaging unit used to capture the plurality of images. Additionally or alternatively, the at least one defined waveband of the temporally modulated illumination may be selected based on an indication of the parameter of the tissue that is to be measured. For example, certain tissue parameters may be measurable under certain lighting conditions such as under light of a particular wavelength.

At step 504, the method 500 comprises generating a composite image 202 based on the plurality of images 200 or a subset of the plurality of images. At step 506, the method 500 comprises generating an ambient light corrected image 204 based on the plurality of images 200. As discussed above, any ambient light correction technique may be employed to perform the ambient light correction in respect of the plurality of images 200. The method 500 comprises, at step 508, measuring, using the ambient light corrected image, a parameter of the tissue. At step 510, the method 500 comprises generating a representation 214 of the tissue based on a combination of the composite image 202 and an indication of the measured parameter. The method 500 comprises, at step 512, providing the generated representation 214 for display. For example, the representation 214 may be provided to the display unit 302 for presentation to the subject.

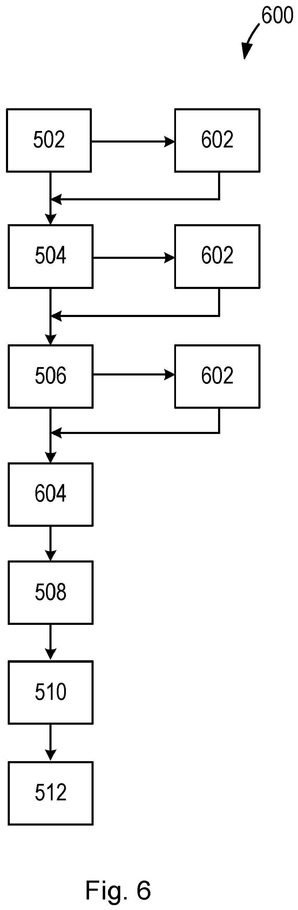

Fig. 6 is a flowchart of a further example of a method 600, which may comprise a computer implemented method for generating image data. The method 600 may comprise one or more steps of the method 500 discussed above. The method 600 may further comprise, at step 602, applying gamma correction processing or inverse-gamma correction processing to at least one of: the plurality of images 200; the composite image 202; and the ambient light corrected image 204. In some examples, some imaging units, such as camera systems, may carry out gamma correction. If gamma correction is applied by a rolling shutter imaging unit, then inverse-gamma correction should be applied to all images before further processing is performed.

At step 604, the method 600 may further comprise generating, based the ambient light corrected image 204, image data representing the tissue in a color corresponding to a defined waveband. For example, image data may be generated that represents the tissue in a red, green or blue color as shown in images 206, 208 and 210, respectively, in Fig. 2. In other examples, image data may be generated that represents the tissue in a color corresponding to defined waveband that is not red, green or blue. Measuring the parameter (step 508) may comprise measuring a parameter using the image data. In other words, the ambient light corrected image 204 may be processed to filter out colors that correspond to wavelengths falling outside of the defined waveband, leaving a version of the ambient light corrected image that has a color corresponding to the defined waveband (e.g. red). The processed version of the ambient light corrected image (i.e. the image data) may then be analyzed to determine the tissue parameter.

Thus, in some embodiments, measuring the parameter of the tissue (step 508) may comprise applying at least one filter to the ambient light corrected image 204 to obtain at least one filtered ambient light corrected image (e.g. the images 206, 208 and 210). Measuring the parameter of the tissue may further comprise determining quantitative information regarding the parameter from the at least one filtered ambient light corrected image.

In some embodiments, the step 510 of generating a representation 214 of the tissue may comprise the step of generating, based on the measured parameter, a parameter image showing an indication of the measured parameter of the tissue; applying a weighting factor to the composite image and the parameter image; and generating a representation of the tissue based on a combination of the weighted composite image and the weighted parameter image the parameter image may comprise an image that shows visually a representation of the parameter or a measure of the parameter, such as the images 212 and 216. The weighting factor applied to the composite image and the parameter image may be selected to achieve a resulting representation that shows clearly the indication of the measured parameter on the composite image of the subject's tissue. For example, to generate the representation 214, a weighting factor of 0.5 has been applied to the composite image 202, and that weighted composite image has been combined with the parameter image 212 to achieve the representation 214. Thus, in this example, a weighting factor of 1 has been applied to the parameter image 212. In other examples, different weighting factors may be applied. The weighting factor may be applied to a pixel value (e.g. a greyscale value or a pixel intensity value for one or more of the red, green and blue channels).

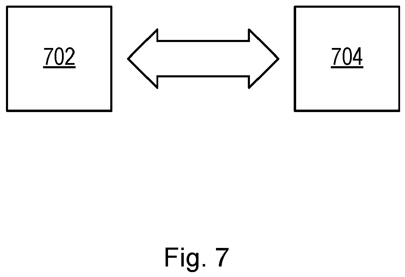

According to fourth aspect, a computer program product is provided. Fig. 7 is a schematic illustration of an example of a processor 702 in communication with a computer-readable medium 704. According to various embodiments, a computer program product comprises a non-transitory computer-readable medium 704, the computer-readable medium having computer-readable code embodied therein, the computer-readable code being configured such that, on execution by a suitable computer or processor 702, the computer or processor is caused to perform steps of the methods 500, 600 disclosed herein. The processor 702 may comprise or be similar to the processor 102 discussed above.

Embodiments disclosed herein provide a mechanism by which analysis of a subject's tissue (e.g. skin) can be performed in a consistent manner, such that varying ambient light conditions when images are captured at different times do not affect the tissue parameter measurement made. The output of the analysis is then displayed to a user (e.g. the subject) on a non-ambient light corrected image, so that the representation that is presented to the user is more realistic (i.e. true to life), meaning that the user is likely to be able to see clearly the measurements of the tissue parameter at different regions the tissue.

The processor 102, 702 can comprise one or more processors, processing units, multi-core processors or modules that are configured or programmed to control components of the system 100, 300 in the manner described herein. In particular implementations, the processor 102, 702 can comprise a plurality of software and/or hardware modules that are each configured to perform, or are for performing, individual or multiple steps of the method described herein.

The term "module", as used herein is intended to include a hardware component, such as a processor or a component of a processor configured to perform a particular function, or a software component, such as a set of instruction data that has a particular function when executed by a processor.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A system (100) for generating image data, the system comprising:
a light source (104) configured to provide temporally modulated illumination to tissue of a subject;
an imaging unit (106) configured to capture, while the tissue is illuminated by the temporally modulated illumination, a plurality of images of the tissue of the subject, wherein each of the plurality of images includes a plurality of bright bands and dark bands, wherein a bright band corresponds to a high state of the temporally modulated illumination and a dark band corresponds to a low state of the temporally modulated illumination; and
a processor (102) configured to:
generate a composite image based on the plurality of images or a subset of the plurality of images generate an ambient light corrected image based on the plurality of images;
measure, using the ambient light corrected image, a parameter of the tissue;
generate a representation of the tissue based on a combination of the composite image and an indication of the measured parameter; and
provide the generated representation for display.

2. A system (100) according to claim 1, wherein the imaging unit is configured to operate in a rolling shutter mode;
wherein the light source is configured to provide temporally modulated illumination in the form of at least one temporally modulated illumination signal having a wavelength falling within a defined waveband;
wherein a frequency of the temporally modulated illumination is not a multiple integral of a frame rate of the imaging unit.

3. A system (100, 300) according to claim 1 or claim 2, further comprising:
a display unit (302) configured to display at least one of: the plurality images; the composite image; the ambient light corrected image; an indication of the measured parameter; and the generated representation.

4. A system (100, 300) according to any of the preceding claims, wherein the light source is configured to generate temporally modulated illumination having a wavelength within a defined waveband; wherein the waveband is selected based on at least one of: an imaging parameter of the imaging unit used to capture the plurality of images; and an indication of the parameter of the tissue that is to be measured.

5. A system (100, 300) according to any of the preceding claims, wherein the processor (102) is configured to measure a parameter of the tissue by:
extracting, from the ambient light corrected image, data corresponding to at least one of a red image channel, a green image channel and a blue image channel; and
determining a physiological parameter of the tissue from at least one of the red image channel, the green image channel and the blue image channel.

6. An interactive mirror (400) comprising:
a reflective component (402); and
the system (100) according to any of the preceding claims.

7. A computer-implemented method (500) for generating image data, the method comprising:
receiving (502) a plurality of images of tissue of a subject illuminated by temporally modulated illumination, wherein each of the plurality of images includes a plurality of bright bands and dark bands, wherein a bright band corresponds to a high state of the pulsed illumination and a dark band corresponds to a low state of the temporally modulated illumination;
generating (504) a composite image based on the plurality of images or a subset of the plurality of images;
generating (506) an ambient light corrected image based on the plurality of images;
measuring (508), using the ambient light corrected image, a parameter of the tissue;
generating (510) a representation of the tissue based on a combination of the composite image and an indication of the measured parameter; and
providing (512) the generated representation for display.

8. A computer-implemented method (500, 600) according to claim 7, further comprising:
generating (604), based the ambient light corrected image, image data representing the tissue in a color corresponding to a defined waveband;
wherein measuring a parameter comprises measuring a parameter using the image data.

9. A computer-implemented method (500, 600) according to claim 7 or claim 8, wherein measuring (508) the parameter of the tissue comprises:
applying at least one filter to the ambient light corrected image to obtain at least one filtered ambient light corrected image; and
determining quantitative information regarding the parameter from the at least one filtered ambient light corrected image.

10. A computer-implemented method (500, 600) according to any of claims 7 to 9, wherein the temporally modulated illumination comprises temporally modulated illumination having a wavelength falling within at least one defined waveband.

11. A computer-implemented method (500, 600) according to claim 10, wherein the at least one defined waveband of the temporally modulated illumination is selected based on a responsivity of an imaging unit used to capture the plurality of images.

12. A computer-implemented method (500, 600) according to claim 10 or claim 11, wherein the at least one defined waveband of the temporally modulated illumination is selected based on an indication of the parameter of the tissue that is to be measured.

13. A computer-implemented method (500, 600) according to any of claims 7 to 12, further comprising:
applying (602) gamma correction processing or inverse-gamma correction processing to at least one of: the plurality of images; the composite image; and the ambient light corrected image.

14. A computer-implemented method (500, 600) according to any of claims 7 to 13, wherein generating (510) a representation of the tissue comprises:
generating, based on the measured parameter, a parameter image showing an indication of the measured parameter of the tissue;
applying a weighting factor to the composite image and the parameter image; and
generating a representation of the tissue based on a combination of the weighted composite image and the weighted parameter image.

15. A computer program product comprising a non-transitory computer-readable medium (704), the computer-readable medium having computer-readable code embodied therein, the computer-readable code being configured such that, on execution by a suitable computer or processor (702), the computer or processor is caused to perform the method of any of claims 7 to 14.
